# EUROPÄISCHE PATENTSCHRIFT

(11) **EP 1 337 625 B1**
(45) Veröffentlichungstag und Bekanntmachung des Hinweises auf die Patenterteilung: **14.07.2004**
(21) Anmeldenummer: 01998624.9
(22) Anmeldetag: 16.11.2001
(51) Int. Cl.: C12N 5/06, C12N 5/00, C12N 5/02, C12N 5/08

(54) **STAMMZELLEN, VERFAHREN ZU DEREN EXPANSION IN VITRO SOWIE DEREN VERWENDUNG**
STEM CELLS, METHOD FOR EXPANSION IN VITRO AND USE THEREOF
CELLULES SOUCHES, PROCEDE D'EXPANSION IN VITRO ET UTILISATION DE CES CELLULES SOUCHES

(30) Priorität: 29.11.2000 DE 10059346; 21.02.2001 DE 10108264
(43) Veröffentlichungstag der Anmeldung: 27.08.2003
(73) Patentinhaber: Scheller, Albert, 73240 Wendlingen (DE); Rader, William, 73240 Wendlingen (DE); Rennebeck, Klaus, 73240 Wendlingen (DE)
(72) Erfinder: Scheller, Albert, 73240 Wendlingen (DE); Rader, William, 73240 Wendlingen (DE); Rennebeck, Klaus, 73240 Wendlingen (DE)
(74) Vertreter: Patentanwälte Hosenthien-Held und Dr. Held
(86) Internationale Anmeldenummer: PCT/EP2001/013329
(87) Internationale Veröffentlichungsnummer: WO 2002/044346

(56) Entgegenhaltungen:
- US-A- 5 409 825
- DE BRUYN ET AL.: "Expansion of purified CD34+ cord blood cells in stromal cell-free long term cultures in presence of recombinant growth factors (Abstract Nr. 1907)" BLOOD, Bd. 82, Nr. 10, 15. November 1999 (1999-11-15), Seite 481a XP001070917

## Beschreibung

Die vorliegende Erfindung betrifft ein Verfahren zur Expansion von Stammzellen in vitro, nach dem Verfahren hergestellte Stammzellen sowie die Verwendung der Stammzellen zur Herstellung eines Arzneimittels.

Stammzellen besitzen die Fähigkeit, sich prinzipiell nahezu unbegrenzt oft zu teilen und dabei spezialisierte Zellen hervorzubringen. Im Verlaufe der natürlichen Entwicklung eines Menschen oder eines anderen Lebewesens treten dabei verschiedene Formen vom Stammzellen auf. Bei der befruchteten Eizelle und den daraus in der ersten Teilung hervorgehenden Zellen handelt es sich um totipotente Zellen, die dadurch gekennzeichnet sind, dass sich aus jeder einzelnen Zelle ein kompletter Organismus entwickeln kann. Wenig später in der Entwicklung, kommt es zur Bildung von pluripotenten Stammzellen. Aus diesen können sich viele Arten von Zellen entwickeln, aber sie haben die Fähigkeit verloren, einen vollständigen Organismus aus einer einzelnen Zelle zu bilden. Diese pluripotenten Zellen unterliegen einer weiteren Spezialisierung in solche Stammzellen, aus denen sich dann Zellen mit speziellen Funktionen entwickeln. Beispiele dafür sind hämatopoetische Stammzellen, die für die Entwicklung von Erytrozyten, Leukozyten und Trombozyten verantwortlich sind, oder Stammzellen der Haut, aus denen sich die verschiedenen Arten vom Hautzellen entwickeln. Diese bereits stärker spezialisierten Stammzellen werden als multipotent bezeichnet. Multipotente Stammzellen befinden sich auch im erwachsenen Organismus.

In den letzten Jahren ist die Forschung über die Isolierung, Vermehrung und die Verwendung insbesondere menschlicher Stammzellen in den Mittelpunkt des Interesses gerückt. Dies hat mehrere Ursachen. Einerseits sind Stammzellen zu einem wichtigen Objekt von Projekten der Grundlagenforschung geworden, die sich mit den komplexen Vorgängen der Zellentwicklung und -differenzierung beschäftigen. Da einige der größten medizinischen Herausforderungen unserer Zeit, wie z.B. Krebserkrankungen, einen abnormen Verlauf der Zellspezialisierung und Zellteilung zur Ursache haben, ermöglicht die Stammzellenforschung ein tieferes Verständnis der zellulären Vorgänge bei diesen Erkrankungen, was zu neuen Therapieansätzen führen kann.

Darüber hinaus führt die Stammzellenforschung zu völlig neuen Möglichkeiten bei der Entwicklung und dem Testen von Medikamenten. So können Stammzellen ein wichtiges Werkzeug bei der Vorselektion von Arzneimitteln auf Wirksamkeit und mögliche Nebenwirkungen darstellen. Nur diejenigen Arzneimittel, die sich im Stammzellen-System als wirksam erwiesen haben und gleichzeitig hinreichend geringe Nebenwirkungen zeigen, werden dann im Tierversuch und/oder in späteren klinischen Untersuchungen eingesetzt. Auf diese Art kann die Entwicklung von Arzneimitteln beschleunigt und gleichzeitig die Anzahl notwendiger Tierversuche reduziert werden.

Die wohl weitreichendsten Möglichkeiten der Verwendung von Stammzellen in der Medizin liegen jedoch in der sogenannten Zelltherapie. Zahlreiche Krankheiten sind die Folge von Störungen der zellulären Funktion oder der Zerstörung von Körpergewebe. Durch die Verwendung von Stammzellen kann es auf vielen Gebieten möglich werden, geschädigte Zellen oder Gewebe zu ersetzen und dadurch eine Vielzahl von Erkrankungen zu behandeln und/oder zu heilen, wie z.B. Krebserkrankungen, Herzerkrankungen, Diabetes, die Parkinson- und die Alzheimer-Krankheit, Verletzungen des Rückenmarkes, Hirnschläge, Verbrennungen, Ostioartritis oder rheumatoide Arthritis. Angesichts der enormen Möglichkeiten, die Stammzellen für die Entwicklung neuer Therapieformen bei vielen Erkrankungen eröffnen könnten, besteht ein großer Bedarf an Verfahren, die ihre Gewinnung, Kultivierung, Vermehrung und weitere Handhabung verbessern.

Da Stammzellen im Organismus nur in relativ geringer Anzahl vorkommen und ihre Gewinnung mit relativ großen Schwierigkeiten verbunden ist, besteht insbesondere ein großer Bedarf an Verfahren zur Kultivierung von Stammzellen in vitro (ex vivo). In vitro bedeutet hier, dass die Zellen außerhalb des Körpers kultiviert werden. Die Kultivierung von Stammzellen in vitro hat sich jedoch bislang als schwierig erwiesen. Ein Grundproblem besteht darin, während der in vitro Kultivierung Bedingungen einzustellen, die die Teilung der Stammzellen stimulieren, ohne dass damit eine Differenzierung und somit ein Verlust der Stammzell-Eigenschaften einhergeht. In der Vergangenheit wurden solche Bedingungen insbesondere durch die Anwesenheit von Stromazellen ermöglicht. Aufgrund der technischen Schwierigkeiten, die mit der Verwendung von Stromazellkulturen verbunden sind, besteht jedoch ein großer Bedarf an Verfahren, die die Kultivierung von Stammzellen in Abwesenheit von Stroma ermöglichen.

Ausgehend von diesem Stand der Technik liegt der vorliegenden Erfindung die Aufgabe zugrunde, ein Verfahren bereitzustellen, das die Expansion von Stammzellen in vitro in möglichst einfacher Weise ermöglicht und die oben beschriebenen Nachteile bisheriger Verfahren vermeidet. Des weiteren sollen vorteilhafte Verwendungen der so hergestellten Stammzellen vorgeschlagen werden.

Erfindungsgemäß wird diese Aufgabe zunächst durch ein Verfahren gelöst, bei dem die Stammzellen während der Expansion kontinuierlich oder zeitweise einem Mikrostrom und/oder einem elektromagnetischem Feld gleichbleibender und/oder wechselnder Polarität ausgesetzt werden. Darüber hinaus wird die Aufgabe durch die nach diesem Verfahren hergestellten Stammzellen und deren Verwendung zur Herstellung eines Arzneimittels gelöst.

Das Verfahren eignet sich für alle Arten von Stammzellen, aus dem menschlichen sowie aus dem tierischen Organismus. Die Verwendung des Mikrostroms bzw. des elektromagnetischen Feldes führt überraschender Weise zu einer Expansion der Stammzellen in stromazellfreier Kultur, bei der die Stammzellen viele Teilungszyklen durchlaufen können, ohne dass eine Differenzierung der Zellen erfolgt. Bevorzugt wird dabei ein Mikrostrom einer Stromstärke von etwa 1 µA bis 100 mA, beispielsweise etwa 10 µA bis 50 mA, insbesondere etwa 25 µA bis 25 mA eingesetzt. Als besonders vorteilhaft hat es sich erwiesen, die kultivierten Stammzellen nur zeitweise dem Mikrostrom auszusetzen. Besonders gute Ergebnisse werden dabei erhalten, wenn man die Stammzellen für bis zu etwa 5 Stunden pro Tag, insbesondere etwa 20 Sekunden bis 5 Stunden pro Tag dem Mikrostrom aussetzt.

Die Expansion der Stammzellen erfolgt vorteilhaft in einem Kulturmedium, dem neben üblichen Bestandteilen hrLIF (rekombinanter humaner Leukämie inhibierender Faktor), insbesondere in einer Konzentration von etwa 1 pM bis 10 µM, besonders bevorzugt etwa 100 pM bis 100 nM, hrFGF (rekombinanter humaner fötaler Wachstumsfaktor), insbesondere in einer Konzentration von etwa 1 pM bis 10 µM, besonders bevorzugt etwa 100 pM bis 100 nM, NGF (Nervenwachstumsfaktor), insbesondere in einer Konzentration von etwa 1 pM bis 10 µM, besonders bevorzugt etwa 100 pM bis 100 nM und/oder MGF (Mesenchymaler Wachstumsfaktor), insbesondere in einer Konzentration von etwa 1 pM bis 10 µM, besonders bevorzugt etwa 100 pM bis 100 nM, zugesetzt werden. Die genannten Bestandteile hrLIF, hrFGF, NGF und/oder MGF weisen vorzugsweise eine Aktivität von etwa 20 bis 70% auf. Weitere bevorzugte Bestandteile des Kulturmediums sind NADPH und/oder Phosphat. Die Kultivierung der Stammzellen erfolgt bevorzugt bei einer Temperatur von etwa 34 bis 42°C, insbesondere 37 bis 40°C, und bei einem pH-Wert von etwa 6,8 bis 7,5, insbesondere von etwa 7,0 bis 7,4.

Ein besonderer Vorteil des Verfahrens besteht darin, dass die kultivierten Stammzellen auch nach vielen Teilungszyklen keiner Differenzierung unterliegen. Um jedoch den undifferenzierten Zustand der Stammzellen nach der Expansion sicherzustellen, ist es bevorzugt, die expandierten Stammzellen bezüglich ihres Differenzierungsgrades einem Test zu unterziehen. Der Differenzierungsgrad wird dabei insbesondere durch die Expression bzw. nicht-Expression von Oberflächenmolekülen definiert. Als besonders vorteilhaft hat es sich erwiesen, den Differenzierungsgrad der Stammzellen nach der Expansion durch einen Test auf Expression von CD117 zu untersuchen. Bei CD117 (c-kit) handelt es sich um ein Transmembranprotein mit Rezeptor Tyrosinkinase Eigenschaften, das als Rezeptor für SLF dient. Zu CD117 siehe auch L. K. Ashman, et al., (1994) J Cellular Physiol 158: 545-554, Leukocyte Typing V (S. F. Schlossman, et al., eds.) Oxford University Press, Oxford, (1995) 1856, 1882-1888, J. R. Keller, et al., (1995) Blood 86: 1757-1764 und Leukocyte Typing VI (T. Kishimoto, et al., eds.) Garland Publishing, Inc., New York (1997) 816-818. Vorteilhaft wird neben dem Test auf den Differenzierungsgrad der expandierten Stammzellen auch ein Test auf Kontamination mit infektiösen Agenzien durchgeführt, insbesondere auf Kontamination mit Mycoplasma incognitus, Hepatitis B Viren, Hepatitis C Viren und/oder HIV Viren.

In einer besonders bevorzugten Ausführungsform der Erfindung werden die Stammzellen vor oder nach der Expansion einer Selektion unterworfen. Das Ziel dieser Selektion besteht darin, bestimmte Populationen der Stammzellen, die für eine spätere Verwendung besonders geeignet sind, von anderen Populationen abzutrennen. Diese Selektion kann insbesondere bei einer späteren Verwendung der Stammzellen zur Behandlung von Patienten von großer Bedeutung sein. Bei einer besonders bevorzugten Ausführungsform erfolgt die Selektion der Stammzellen bezüglich GP41, HIV nef, CCR5 und/oder CXCR4.

Bei GP41 handelt es sich um ein virales Protein aus dem HIV-Virus, das für den Eintritt des HIV-Virus in die Zielzelle von Bedeutung ist. Zu GP41 siehe auch Chan, D. C. , Fass, D., Berger, J. M., and Kim, P. S. (1997). Core structure of gp41 from the HIV envelope glycoprotein.: *Cell* : **89** : 263-73; Chang, D. K., Cheng, S. F., and Chien, W. J. (1997). The amino-terminal fusion domain peptide of human immunodeficiency virus type 1 gp41 inserts into the sodium dodecyl sulfate micrelle primarily as a helix with a conserved glycerine at the micelle-water-interface.: *J. Virol* **71**: 6593-603 und Chen, S. S. (1994). Functional role of the zipper motif region of human immunodeficiency virus type 1 transmembrane protein gp41.: *J. Virol* **68**: 2002-10. Für die Infektion von Zellen mit dem HIV-Virus ist auch das Protein HIV-nef von Bedeutung. HIV-nef ist ein regulatorisches Protein des HIV-Virus, das eine inhibitorische Wirkung auf die Immunabwehr des Zielorganismus ausübt und dadurch die HIV-Replikation erleichtert. Nähere Informationen zu HIV-nef finden sich in Aiken, C., Konner, J., Landau, N. R., Lenburg, M. E., and Trono, D. (1994). Nef induces CD4 endocytosis: requirement for a critical dileucine motif in the membrane-proximal CD4 cytoplasmic domain. *Cell* **76**, 853-64; Aiken C., und Trono, D. (1995). Nef stimulates human immunodeficiency type 1 proviral DANN synthesis. *J Virol* **69**, 5048-56; Baur, A. S., Sawai, E. T., Dazin, P., Fantl, W. J., Cheng, M. C , and Peterlin, B. M. (1994). HIV-1 Nef leads to inhibition or activation of T cells depending on its intracellular localization. *Immunity* **1**, 373-84 und Collins, K. L., Chen, B. K., Kalams, S. A., Walker, B. D., and Baltimore, D. (1998). HIV-1 Nef protein protects infected primary cells against killing by cytotoxic T lymphocytes. *Nature* **391**, 397-401. Bei CCR5 und CXCR4 handelt es sich um Chemokinrezeptoren aus der Chemokinrezeptorfamilie, siehe hierzu auch Samson et al. *Biochemistry* 1996 35:3362-3367; Doranz et al. *Cell* 1996 85:1149-1158; Samson et al. *Nature* 1996 382:722-725; Rucker et al. *Cell* 1996 87:437-446; Bleul, C. C.; Wu, L.; Hoxie, J. A.; Springer, T. A.; Mackay, C. R.; The HIV Coreceptors CXCR4 and CCR5 are differentially expressed and regulated on human T lymphocytes. Proc Natl Acad Sci USA **94(5)**: 1925-1930 (1997); Di Marzio, P.; Tse, J.; Landau, N. R., Chemokine receptor regulation and HIV type 1 tropism in monocyte-macrophages. AIDS Res Hum Retroviruses 14(2): 129-138 (1998); D'Souza, M. P.; Harden, V. A.; Chemokines and HIV-1 second rezeptors. Nature Medicine 2: 1293-1300 (1996) und Gupta, S. K.; Lysko, P. G.; Pillarisetti, K.; Ohlstein, E.; Stadel, J. M.; Chemokine receptors in human endothelial cells - Functional expression of CXCR4 and its transcriptional regulation by inflammatory cytokines. J Biol Chem 273(7): 4282-4287 (1998).

Als Ausgangs-Stammzellen für das erfindungsgemäße Verfahren eignen sich alle Arten von Stammzellen. Die Stammzellen können dabei nach den im Stand der Technik bekannten Verfahren gewonnen werden. Wenn die Stammzellen nach der Expansion zur Herstellung eines Arzneimittels dienen sollen, können die als Ausgangsmaterial verwendeten Stammzellen aus dem zu behandelnden Patienten selbst oder aber aus einer anderen Person stammen. Nach dem oben beschriebenen Verfahren hergestellte Stammzellen sind ebenfalls Gegenstand der Erfindung, insbesondere Stammzellen, die GP41 negativ, HIV nef negativ, CCR5 negativ und/oder CXCR4 negativ sind.

Ein weiterer Gegenstand der Erfindung besteht in der Verwendung dieser Stammzellen zur Herstellung eines Arzneimittels. Das so hergestellte Arzneimittel umfaßt zur Behandlung eines Patienten vorzugsweise etwa 1 x 10⁵ bis 1 x 10⁹, insbesondere 1 x 10⁶ bis 50 x 10⁶, homozygote und/oder hetero homozygote Stammzellen. Besonders vorteilhafte Ergebnisse werden erzielt, wenn zusätzlich zu den Stammzellen dendritische Zellen verwendet werden. Die dendritischen Zellen können dabei nach einem im Stand der Technik bekannten Verfahren gewonnen werden. Sie können aus dem zu behandelnden Patienten selbst oder aus einer anderen Person stammen. Eine bevorzugte Verwendung liegt dabei in der Herstellung eines Arzneimittels zur Reduzierung des T4/T8 Quotienten. Besonders bevorzugt ist die Herstellung eines Arzneimittels zur Behandlung von HIV.

Das vorliegende Verfahren eignet sich zur Expansion von Stammzellen in vitro. Es wurde gefunden, dass nach dem erfindungsgemäßen Verfahren expandierte Stammzellen viele Teilungszyklen durchlaufen können, ohne dass eine Differenzierung der Zellen eintritt. Das Verfahren läßt sich mit relativ geringem apparativen Aufwand kostengünstig durchführen. Ein weiterer Vorteil des Verfahrens liegt darin, dass es ohne die Verwendung von Stromazellen auskommt. Die so gewonnenen Stammzellen eignen sich sehr gut zur Herstellung eines Arzneimittels, insbesondere zur Behandlung von HIV.

Die Erfindung wird durch die nachfolgenden Beispiele noch näher erläutert:

### Beispiel 1: Expansion von Stammzellen in vitro

### Reagenzien und Materialien:

**1.** Rekombinanter humaner basischer Fibroblasten Wachstumsfaktor (hrFGF) (0,1 µM)
**2.** humaner Leukämie inhibierender Faktor (hrFGF) (0,1 µM)
**3.** mesenchymaler Wachstumsfaktor (MGF) (0,1 µM)
**4.** nicht-essentielle Aminosäuren (200 mM)
**5.** L-Glutamin (200 mM)
**6.** 0,1 mM Mercaptoethanol
**7.** 1 mM Natriumpyruvat
**8.** 20% fötales bovines Serum (FBS)
**9.** DMSO
**10.** Basalmedium:
   i) supplementiertes DMEM Medium (MSCBM), 440 ml
   ii) spezifisches fötales bovines Serum (MCGS), 50 ml
   iii) L-Glutamin 200 mM, 10 ml
   iv) Penicillin-25 Einheiten und Streptomycin 25 µg - in 0,5 ml
**11.** 75 mm #2 Corning Zellkulturgefäß mit 0,2 µm Poren (steril)
**12.** Nalgene® Einwegfiltereinheiten (steril)
**13.** 50 ml Steckkappenzentrifuge (steril)
**14.** Mikrostromgenerator
**15.** Kühlsystem zum Gefrieren durch kontrolliertes Abkühlen, Model 9000 von Gordinier Electronics, Inc.

Herstellung des Basalmediums:
**a)** Die Oberfläche der Reagenzflaschen wurde mit 70 % Isopropanol dekontaminiert
**b)** 50 ml MCGS wurden unter sterilen Bedingungen zu 440 ml MSCBM zugegeben
**c)** zu der im Schritt **b)** hergestellten MSCBM/MCGS-Lösung wurden unter sterilen Bedingungen 10 ml 200 mM L-Glutamin und 0,5 mL Pen/Strep (Penicillin - 25 Einheiten, Streptomycin 25 µg) zugegeben.

Zu dem Basalmedium wurde folgendes zugegeben:
**a)** 10 ml hrLIF/hrFGF (je 0,05 µM)
**b)** 15 ml MGF (0,1 µM)
**c)** 8,75 ml Lösung nicht-essentieller Aminosäuren (200 mM)
**d)** 25 ml L-Glutamin (200 mM)
**e)** 750 µl 0,1 mM Mercaptoethanol
**f)** 1 ml 1 mM Natriumpyruvat
**g)** 73 ml FBS

Anschließend wurden folgende Schritte durchgeführt:
**1.** Das oben hergestellte Medium wurde durch einen Nalgene® Einwegsterilfilter mit einer Porengröße von 0,2 µm steril filtriert.
**2.** Jeweils 20 ml des Mediums wurden unter sterilen Bedingungen in ein Corning 75 mm #2 Kulturgefäß eingefüllt.
**3.** Dem Kulturgefäß wurden unter sterilen Bedingungen etwa 10⁶ menschliche Stammzellen zugegeben.
**4.** Die Kolben wurden vorsichtig auf ihre Rückseite gelegt.
**5.** Die negative und die positive Elektrode des Mikrostromgenerator-Instruments wurden an den gegenüberliegenden Seiten des Kulturgefäß befestigt.
**6.** Die Kolben mit den Elektroden wurden in einem Zellkulturinkubator gestellt, der auf 37°C und 5% CO₂ eingestellt war.
**7.** Anschließend wurde für eine Stunde ein Mikrostrom von 1 mA durch die Zellkulturkolben geleitet.
**8.** In den nächsten 5 Tagen wurden die Zellen weiter inkubiert, wobei jeden Tage für eine Stunde ein Mikrostrom von 1 mA durch die Zellkulturgefäße geleitet wurde.
**9.** Danach wurden die Zellen unter sterilen Bedingungen von der Rückseite des Zellkulturgefäßes abgekratzt.
**10.** Der gesamte Inhalt des Zellkulturgefäßes wurde unter sterilen Bedingungen in ein 50 ml Röhrchen abgesaugt und für etwa 10 min bei etwa 80 g zentrifugiert.
**11.** Der Mediumüberstand wurde unter sterilen Bedingungen abgesaugt, wobei die humanen Stammzellen als Pellet im Röhrchen verblieben.
**12.** Das Zellpellet wurde unter sterilen Bedingungen mit 20 ml 10% DMSO-Phosphatpuffer versetzt und es wurde vorsichtig gemischt.
**13.** Die in Schritt **12.** gewonnene Zellsuspension wurde in 1,5 ml Portionen aliquotiert.
**14.** Die Zellsuspension wurde unter Verwendung des Kühlsystems zum kontrollierten Einfrieren eingefroren.
**15.** Die Lagerung erfolgte bei -15°C.

### Beispiel 2: Behandlung eines an AIDS Erkrankten mit den in Beispiel 1 gewonnenen Stammzellen

Ein HIV-positiver und an AIDS erkrankter Patient wurde intravenös mit 10 x 10⁶ homozygoten und hetero homozygoten Stammzellen behandelt, die nach einem Verfahren wie in Beispiel 1 beschrieben expandiert worden waren und die CCR5 negativ waren.

Im Verlaufe der Behandlung ergab sich ein stark verbessertes Blutbild, wie durch die nachfolgende Tabelle 1 dokumentiert wird:

**Tabelle 1**

| Indikator | Normalwert für gesunde Personen | nach der Stammzellenbehandlung | vor der Stammzellenbehandlung |
|---|---|---|---|
| Lymphozyten absolut | (1000-3500) | 1612 | 1322 |
| Nat. Killerzellen % | (5-20) | 4,3 | 8,0 |
| Nat. Killerzellen abs. | (50-700) | 69 | 106 |
| B-Lymphozyten | (7-23) | 8,8 | 4,0 |
| B-Lymphozyten abs. | (70-805) | 143 | 52 |
| T-Lymphozyten | (60-81) | 85,6 | 86,9 |
| T-Lymphozyten abs. | (600-2800) | 1380 | 1149 |
| T-Helfer/Inducer-Lym. | (32-52) | 38,7 | 26,1 |
| T-Helfer/Inducer-Lym.abs. | (192-1450) | 624 | 346 |
| T8-Suppressor (CD8) | (20-40) | 41,7 | 55,0 |
| T-Suppressor abs. | (120-1120) | 672 | 727 |
| T4/T8-Quotient | (1,4-2,4) | 0,9 | 0,5 |
| HLA-DR pos. | (2-12) | 20 | 37,2 |
| HLA-DR pos. abs. | (12-336) | 322 | 493 |
| Interleukin-2 pos. | (< 35) | 3,7 | 4,4 |
| Interleukin-2 pos. abs. | (< 980) | 59 | 58 |
| akt. T8-Z. HLA-DR pos. | (< 11) | 16,9 | 33,7 |
| akt. T8-HLA-DR pos. abs. | (< 310) | 273 | 446 |
| Interleukin-2 pos. | (< 11) | 2,4 | 3,3 |
| akt. T4-Z. IL2 pos. abs. | ( ) | 39 | 43 |
| T8-Suppr. aktiv | ( ) | 80,3 | 84,8 |
| T8-Suppr. cytotox. akt. | ( ) | 12,1 | 11,0 |
| FT3 | (2,6-5,1) | 2,9 | 4,5 |
| FT4 | (0,7-2,0) | 0,9 | 1,3 |
| TSH | (0,2-3,9) | 1,73 | 1,73 |

## Patentansprüche

1. Verfahren zur Expansion von Stammzellen in vitro, **dadurch gekennzeichnet, dass** die Stammzellen während der Expansion kontinuierlich oder zeitweise einem Mikrostrom und/oder einem elektromagnetischen Feld gleichbleibender und/oder wechselnder Polarität ausgesetzt werden, wobei keine Stromazellen verwendet werden.

2. Verfahren nach Anspruch 1, **dadurch gekennzeichnet, dass** ein Mikrostrom von etwa 1 µA bis 100 mA verwendet wird.

3. Verfahren nach einem der Ansprüche 1 oder 2, **dadurch gekennzeichnet, dass** die Stammzellen für bis zu etwa 5 Stunden pro Tag dem Mikrostrom ausgesetzt werden.

4. Verfahren nach Anspruch 3, **dadurch gekennzeichnet, dass** die Stammzellen für etwa 20 Sekunden bis 5 Stunden pro Tag dem Mikrostrom ausgesetzt werden.

5. Verfahren nach mindestens einem der vorhergehenden Ansprüche, **dadurch gekennzeichnet, dass** die Expansion in einem Kulturmedium durchgeführt wird, dem hrLIF, hrFGF, NGF und/oder MGF zugesetzt wird.

6. Verfahren nach Anspruch 5, **dadurch gekennzeichnet, dass** dem Kulturmedium NADPH und/oder ein Phosphat zugesetzt wird.

7. Verfahren nach mindestens einem der vorhergehenden Ansprüche, **dadurch gekennzeichnet, dass** die Expansion bei einer Temperatur von etwa 34 bis 42°C durchgeführt wird.

8. Verfahren nach mindestens einem der vorhergehenden Ansprüche, **dadurch gekennzeichnet, dass** die Expansion bei einem pH zwischen etwa 6,8 und 7,5 durchgeführt wird.

9. Verfahren nach mindestens einem der vorhergehenden Ansprüche, **dadurch gekennzeichnet, dass** nach der Expansion der Stammzellen ein Test auf den undifferenzierten Zustand der expandierten Stammzellen und/oder auf Kontamination mit infektiösen Agentien durchgeführt wird.

10. Verfahren nach Anspruch 9, **dadurch gekennzeichnet, dass** ein Test auf Expression von CD117 durchgeführt wird.

11. Verfahren nach Anspruch 9 oder 10, **dadurch gekennzeichnet, dass** ein Test auf Kontamination mit Mycoplasma incognitus, Hepatitis B Viren, Hepatitis C Viren und/oder HIV Viren durchgeführt wird.

12. Verfahren nach mindestens einem der vorhergehenden Ansprüche, **dadurch gekennzeichnet, dass** die Stammzellen vor oder nach der Expansion einer Selektion unterworfen werden.

13. Verfahren nach Anspruch 12, **dadurch gekennzeichnet, dass** die Stammzellen einer Selektion bezüglich GP41, HIV nef, CCR5 und /oder CXCR4 unterzogen werden.

## Claims

1. Procedure for expanding stem cells *in vitro,* **characterized in that** the stem cells, during their expansion, are exposed continuously or for a specific period to a microcurrent and/or an electromagnetic field of direct and/or alternating polarity, without any stroma cells being used.

2. Procedure according to Claim 1, **characterized in that** a microcurrent of approx. 1 µA to 100 mA is used.

3. Procedure according to one of Claims 1 or 2, **characterized in that** the stem cells are exposed to the microcurrent for up to approx. 5 hours per day.

4. Procedure according to Claim 3, **characterized in that** the stem cells are exposed to the microcurrent for approx. 20 seconds to 5 hours per day.

5. Procedure according to at least one of the preceding claims, **characterized in that** the expansion is carried out in a culture medium to which hrLIF, hrFGF, NGF and/or MGF are added.

6. Procedure according to Claim 5, **characterized in that** NADPH and/or a phosphate are added to the culture medium.

7. Procedure according to at least one of the preceding claims, **characterized in that** the expansion is carried out at a temperature of approx. 34°C to 42°C.

8. Procedure according to at least one of the preceding claims, **characterized in that** the expansion is carried out at a pH of between 6.8 and 7.5.

9. Procedure according to at least one of the preceding claims, **characterized in that**, following the expansion of the stem cells, a test is carried out to determine the undifferentiated status of the expanded stem cells and/or contamination with infectious agents.

10. Procedure according to Claim 9, **characterized in that** a test is performed to determine the expression of CD117.

11. Procedure according to Claim 9 or 10, **characterized in that** a test is performed for contamination with mycoplasma incognitus, Hepatitis B viruses, Hepatitis C viruses and/or HIV viruses.

12. Procedure according to at least one of the preceding claims, **characterized in that** the stem cells, before or after expansion, are subjected to selection.

13. Procedure according to Claim 12, **characterized in that** the stem cells are subjected to selection with respect to GP41, HIV Nef, CCR5 and/or CXCR4.

## Revendications

1. Procédé pour l'expansion de cellules souche *in vitro,* **caractérisé en ce que** les cellules souche, pendant leur expansion, sont exposés, de façon continue ou pour des périodes spécifiques, à un micro-courant et/ou à un champ électromagnétique à polarité directe et/ou alternante, sans utilisation de cellules stromales.

2. Procédé selon la revendication 1, **caractérisé en ce qu'**on utilise un micro-courant d'environ 1 µA à 100 mA.

3. Procédé selon l'une des revendications 1 ou 2, **caractérisé en ce que** les cellules souche sont exposés au micro-courant pendant une période allant jusqu'à environ 5 heures par jour.

4. Procédé selon la revendication 3, **caractérisé en ce que** les cellules souche sont exposés au micro-courant pendant environ 20 secondes à 5 heures par jour.

5. Procédé selon au moins une des revendications précédentes, **caractérisé en ce que** l'expansion s'effectue dans un milieu de culture auquel sont ajoutés le facteur recombinant humain d'inhibition de la leucémie (hrLIF), le facteur recombinant humain de la croissance fétale (hrFGF), le facteur de la croissance neuronale (NGF) et/ou le facteur de la croissance mésenchymateuse (MGF)

6. Procédé selon la revendication 5, **caractérisé en ce que** le phosphate nicotinamide adénine dinucléotide (NADPH) et/ou un phosphate sont ajoutés au milieu de culture.

7. Procédé selon au moins une des revendications précédentes, **caractérisé en ce que** l'expansion se fait à une température d'environ 34°C à 42°C.

8. Procédé selon au moins une des revendications précédentes, **caractérisé en ce que** l'expansion se fait à un pH entre 6.8 et 7.5.

9. Procédé selon au moins une des revendications précédentes, **caractérisé en ce que**, suivant l'expansion des cellules souche, on effectue un test pour déterminer la non différenciation des cellules souche multipliées et/ou la contamination avec des agents infectieux.

10. Procédé selon la revendication 9, **caractérisé en ce qu'**un test est effectué pour déterminer l'expression du CD117.

11. Procédé selon la revendication 9 or 10, **caractérisé en ce qu'**un test est effectué pour déterminer la contamination avec *mycoplasma incognitus,* des virus de l'hépatite B, virus de l'hépatite C et/ou des virus du SIDA.

12. Procédé selon au moins une des revendications précédentes, **caractérisé en ce que** les cellules souche, avant et après l'expansion, sont soumis à une sélection.

13. Procédé selon la revendication 12, **caractérisé en ce que** les cellules souche sont soumises à une sélection par rapport au GP41, HIV Nef, CCR5 et/ou au CXCR4.
